Europäisches Patentamt

European Patent Office

Office européen des brevets

(19)

(11) Publication number: **0 013 394**
**B1**

(12) **EUROPEAN PATENT SPECIFICATION**

(45) Date of publication of patent specification: **17.08.83**

(51) Int. Cl.³: **C 12 N 5/00, A 61 K 35/36**

(21) Application number: **79105271.5**

(22) Date of filing: **19.12.79**

(54) Process for growing human epidermal cells in tissue culture.

(30) Priority: **02.01.79 US 749**

(43) Date of publication of application:
**23.07.80 Bulletin 80/15**

(45) Publication of the grant of the patent:
**17.08.83 Bulletin 83/33**

(84) Designated Contracting States:
**DE FR GB IT**

(56) References cited:
**US - A - 4 016 036**

**CHEMICAL ABSTRACTS, vol. 90. no. 3, 15th
January 1979, page 394, no. 19988h
Columbus, Ohio, U.S.A. C. L. MARCELO et al.:
"Stratification, specialization, and proliferation
of primary keratinocyte cultures. Evidence of a
functioning in vitro epidermal cell system"
CHEMICAL ABSTRACTS, vol. 89, no. 15, 9th
October 1978, page 386, no. 126973a
Columbus, Ohio, U.S.A. S. H. LIU et al.:
"Isolation and growth of adult human
epidermal keratinocytes in cell culture"**

(73) Proprietor: **Sloan-Kettering Institute For Cancer
Research
1275 York Avenue
New York New York 10021 (US)**

(72) Inventor: **Eisinger, Magdalena G.
30 Pine Terrace
Demarest, New Jersey 07627 (US)**
Inventor: **Hefton, John M.
1 Fifth Avenue
New York, N.Y. 10003 (US)**

(74) Representative: **Schulze Horn, Stefan, Dipl.-Ing.
et al,
Patentanwälte Dipl.-Ing. S. Schulze Horn M.SC.
Dr. H. Hoffmeister Goldstrasse 36
D-4400 Münster (DE)**

(56) References cited:
**CHEMICAL ABSTRACTS, vol. 87, no. 21, 21th
November 1977, page 330, no. 164978x
Columbus, Ohio, U.S.A. V. J. W. LONG:
"Incorporation of 1-14C-acetate into epidermal
lipids. A comparison between isolated
epidermal cells and epidermal slices"**

## Process for growing human epidermal cells in tissue culture

This invention relates to a process for growing human epidermal cells in tissue culture, said process comprising the following steps:

(a) separating the epidermis in human skin from the dermis,
(b) dissociating the epidermis into epidermal cells and suspending the cells in a tissue culture medium, mainly consisting of minimum essential medium according to EAGLE, optionally suitably supplemented with fetal mammal serum,
(c) growing the epidermal cells by incubation at a suitable temperature.

The steps mentioned have been suggested by Kitano et al. (Biochemistry of Cutaneous Epidermal Differentiation, Ed. By Seiji et al, University Park Press, 1977, pp. 319—335). The publication of Kitano et al contains no information of the pH-range used, but mentions, that the tissue growing medim consisted of EAGLE's minimum essential medium was supplemented with 30% fetal bovin serum.

This medium is state of the art (Science, Vol. 130; 1959; pp. 432—437). Use and range of pH is explained in a comprehensive publication by EAGLE (J. Cellular Physiology, Vol. 82, No. 1, 1973, pp. 1—8). The latter is concerned with the optimum pH for the growth of cells, which lies for human cells between pH 6.3—8.5, according to table 1 of said latter EAGLE-publication.

It is therefore the teaching of the combined references, that the pH of the culture medium according to Kitano has to be in the range of pH 6.3—8.4, in spite of the fact, that Kitano is silent about this important parameter. However, in this range the technique of Kitano is not reproducible.

The object of the invention therefore is to give ways and means to find a reproducible and working process for growing human epidermal cells in a tissue culture.

The solution is, using the steps already mentioned and using a tissue culture medium consisting of the minimum essential medium according to EAGLE, optionally suitably supplemented with fetal mammal serum, that the culture has a pH from 5.6 to 5.8.

The inventors found that by departing from the teaching of said combined references, epidermal cells can be grown in a culture medium, or, with other words, the determination of the pH-range was the difference between success and earlier failure from the following prior teaching for culturing tissue cells.

In summary, this invention provides a method for growing human epidermal cells *in vitro* in the absence of dermal components to produce single or multi-layered pure human epidermis, which can be used directly for example in the treatment of burn victims.

The epidermis of human skin can be separated from the dermis using the Medawar trypsin technique (*Nature,* Vol. *148*, 783—4 (1941)).

The separated epidermis is dissociated into individual epidermal cells mechanically and the cells are then seeded into the suitable tissue culture medium at the required pH. It is preferred to remove any remaining clumps or pieces of tissue, for example, by filtration, prior to seeding. This removes non-viable cells that otherwise interfere with growth of the epidermal cells.

The suitable tissue culture medium consists of minimum essential medium according to EAGLE, which is optionally suitable supplemented with fetal mammal serum, and has a pH in the range of 5.6—5.8 and supports and promotes the growth of the epidermal cells. The growing is preferably carried out in a plastic tissue culture vessel, but other cell growth techniques can be used.

A preferred culture medium contains from 5—15% by volume, preferably about 10%, fetal calf serum which is commercially available from a number of sources. Hydrocortisone can be added to the medium to make the colony morphology more orderly and distinctive. Rheinwald and Green, *Cell, 6,* 331—44 (1975). It is also desirable to reduce the amount of fetal calf serum to about 5% once the cells become attached to the vessel.

Seeding of the epidermal cells in the culture medium is preferably in the range of about 5—8×10$^5$ cells/ml which has been found to promote optimal growth of the epidermis.

Growth in the medium is preferably carried out at a temperature of about 35—36°C under relative humidity of about 80% in the presence of 5% carbon dioxide and 95% atmospheric air.

To apply or transfer the single or multi-layered epidermal sheet grown *in vitro* according to the invention, a transfer member such as the dermal side of pig skin or a collagen sponge is used. The epidermal sheet becomes sufficiently adhered to the transfer member (after the edges of the sheet are cut from the tissue vessel with sharp scalpel) so that it can be removed from the tissue vessel and applied, for example, to an afflicted area on a burn victim. To facilitate host acceptance, the skin of the burn victim is used but this is not essential.

The invention is notable for the absence of any dermal components which have previously been thought to be essential for human epidermal cell growth *in vitro*. This system offers an opportunity to grow human epidermal cells which have retained the potential to differentiate into multilayered structures. These, in turn, can be used for transplantation mainly in cases of burn patients, where rapid enlargement of the epidermis is necessary to cover the affected area. Because of the absence of any dermal components, these cells can be also used for testing of the effects of different drugs and/or carcinogens on the human epidermis. Thus the invention finds use for treatment of burn patients either as autografts of homografts, for the testing of drugs and/or carcinogens, for the growth of other epidermal cells such as conjunctival epidermis of corneal cells and for the growth of viruses which require epidermal cells which differentiate, such as the human wart virus.

The addition of the epidermal growth factor, and/or of specific nutrients required by the epidermal cells can be used for continued *in vitro* survival.

The following example is intended to illustrate the invention without limiting same. Abbreviations used are as follows:

U PNC —units of penicillin
STM —streptomycin
ATV —(MADIN, S. H. and DARBY, N. B., Jr.: Established cell lines of normal and adult bovine and bovine origin. Proc. Soc. Exp. Biol. Med. 98:574—576 (1958).) NaCl, 8 gm/l; KCl, 0.4 gm; dextrose, 1 gm; $NaHCO_3$, 0.58 gm; trypsin (DIFCO 1:250), 0.5 gm; versene (disodium salt), 0.2 gm; distilled water to 1000 ml.
EDTA —Ethylendiamine tetraacetic acid, disodium salt (Sigma).
MEM —Minimum Essential Medium (Eagle) with Earle's Salts.
GMEM —10% Fetal calf serum (FCS), 100 U PNC/ml, 100 g STM/ml, 0.6 mcg Fungizone/ml, 0.5 mcg hydrocortison/ml, L-glutamine 200 mM 0.292 g/ml, and MEM—non-essential amino acids 10 mM (Grand Island Biol. Co.) 1 ml/100 ml.

Full thickness or split thickness human skin can be used. The skin can be obtained from any part of the body, including foreskin. If full thickness skin is used, the dermis should be trimmed down as far as possible. The skin is washed in MEM with 1,000 U PNC/ml, 1,000 mcg/ml STM and 3.00 mcg/ml Fungizone for 30 min., followed by two washes for 10 min. each in the same medium. Two additional washes 10 min. each are in MEM with 10 times less antibiotics than above. The skin is then cut into 4 mm² pieces and immersed into a 0.02% EDTA (prepared in distilled $H_2O$) for 10—15 min. Afterwards, the pieces are transferred to a 0.25% trypsin (Trypsin 1:250 "Difco" certified) prepared in phosphate buffered saline, without $Ca^{++}$ and $Mg^{++}$. The pieces immersed in this solution are kept at 4°C for 16—18 hr. Using fine forceps, the epidermis is carefully separated from the dermis. Dermis is discarded (or used for growth of fibroblasts) and the epidermis is floated in a solution of ATV. When all epidermis is collected, the epidermal cells are separated from each other by mechanical teasing with forceps. The cells eluted into the supernatant are transferred into a new dish and further separated by vigorous pipetting with a pasteur pipette. The single cell suspension (checked under a microscope) is collected into fetal calf serum, removing any remaining clumps or pieces of tissue by filtration through 4 layers of sterile gauze mesh. To the remaining epidermal tissue, fresh ATV is added and the procedure as described above is repeated until all tissue is completely disaggregated. The cells collected in fetal calf serum are centrifuged at 1,000 rpm for 10 min., the supernatant discarded and the cells resuspended in GMEM, at pH 5.6—5.8 are counted. The viability of such cultures is usually approximately 90%. The cells are seeded at 5—8×10⁵ cells/ml into plastic tissue culture vessels. GMEM is used for the growth of epidermal cells. The cells are grown in the presence of 5% $CO_2$ and 95% air, at 80% humidity and 35—36°C.

## Claims

1. Process for growing human epidermal cells in tissue culture, said process comprising the following steps:

(a) separating the epidermis in human skin from the dermis,
(b) dissociating the epidermis into epidermal cells and suspending the cells in a tissue culture medium, mainly consisting of minimum essential medium according to EAGLE, optionally suitably supplemented with fetal mammal serum,
(c) growing the epidermal cells by incubation at a suitable temperature,

characterized by that the tissue culture medium has a pH of from 5.6 to 5.8.

2. Process of claim 1, characterized by that the culture medium contains from 5 to 15 per cent by volume fetal calf serum.

3. Process of claim 2, characterized by that the culture medium contains about 10% by volume fetal calf serum and the amount of fetal calf serum in the culture medium is reduced to about 5% by volume after the cells become attached to a culturing vessel.

4. Process of claim 1, wherein the cells are grown at a temperature of 35°—36°C.

5. Process of claim 1, characterized by that the epidermal cells are seeded in the culture medium at a density of from 5 to 8×10⁵ cells/ml.

6. Process of claim 1, characterized by that the epidermal cells are grown into a epidermal sheet in a tissue culture vessel which is removed from the wall of the vessel by adhering the sheet to a transfer member.

7. Process of claim 6, characterized by that the epidermal sheet is adhered to the dermal side of pig skin.

**Patentansprüche**

·1. Verfahren zur Kultivierung von menschlichen Epidermis-Zellen, das folgende Verfahrensschritte umfaßt:

(a) Trennung der Epidermis der meschlichen Haut von der Dermis,

(b) Auftrennung der Epidermis in Epidermis-Zellen und Suspension der Zellen in einem Gewebe-Kultivationsmedium, das haptsächlich aus "minimum essential medium" nach EAGLE besteht, das gegebenenfalls in geeigneter Weise durch fetales Säugetierserum ergänzt ist und

(c) Kultivieren und Wachsenlassen der Epidermis-Zellen durch Inkubation bei einer geeigneten Temperatur,

dadurch gekennzeichnet, daß das Gewebe-Kultivationsmedium einen pH-Wert zwischen 5,6 und 5,8 aufweist.

2. Verfahren nach Anspruch 1, dadurch gekennzeichnet, daß das Kultivationsmedium 5 bis 15 Vol.-% fetales Kalbsserum enthält.

3. Verfahren nach Anspruch 2, dadurch gekennzeichnet, daß das Kultivationsmedium etwa 10 Vol.-% fetales Kalbsserum enthält, und daß der Gehalt an fetalem Kalbsserum in dem Medium auf 5 Vol.-% reduziert wird, nachdem die Zellen am Kultivationsgefäß anhaften.

4. Verfahren nach Anspruch 1, dadurch gekennzeichnet, daß die Zellen bei einer Temperatur zwischen 35° bis 36°C kultiviert werden.

5. Verfahren nach Anspruch 1, dadurch gekennzeichnet, daß die Epidermis-Zellen in dem Kultivationsmedium nach der Suspension in einer Besetzung von 5 bis $8 \cdot 10^5$ Zellen/ml vorliegen.

6. Verfahren nach Anspruch 1, dadurch gekennzeichnet, daß die Epidermis-Zellen in einem Kultivationsgefäß zu einem Epidermis-Blatt oder -Pflaster wachsen, und daß die Ablösung des Blattes oder Pflasters von der Gefäßwand durch ein Überführungselement erfolgt, an dem es anhaftet.

7. Verfahren nach Anspruch 6, dadurch gekennzeichnet, daß das Epidermis-Blatt oder -Pflaster zum Anhaften an der Dermis-Seite einer Schweinehaut gebracht wird.

**Revendications**

1. Procédé pour faire croître des cellules épidermiques humaines dans une culture de tissus, ce procédé comprenant les stades suivants:

a) séparation de l'épiderme de la peau humaine du derme,

b) dissociation de l'épiderme en cellules épidermiques et mise en suspension des cellules dans un milieu de culture de tissus, constitué principalement de milieu essential minimum selon EAGLE, convenablement supplémenté si on le désire avec du sérum de foetus de mammifère,

c) culture de cellules épidermiques par incubation à une température appropriée,

caractérisé en ce que le milieu de culture de tissus a un pH de 5,6 à 5,8.

2. Procédé de la revendication 1, caractérisé en ce que le milieu de culture contient de 5 à 15% en volume de sérum de foetus de veau.

3. Procédé de la revendication 2, caractérisé en ce que le milieu de culture contient environ 10% en volume de sérum de foetus de veau et en ce que la quantité de sérum de foetus de veau dans le milieu de culture est réduite à environ 5% en volume après que les cellules se sont fixées au récipient de culture.

4. Procédé suivant la revendication 1, caractérisé en ce que les cellules sont cultivées à une température de 35 à 36°C.

5. Procédé suivant la revendication 1, caractérisé en ce que le milieu de culture est ensemencé avec les cellules épidermiques à raison de 5 à $8 \times 10^5$ cellules/ml.

6. Procédé suivant la revendication 1, caractérisé en ce que les cellules épidermiques grandissent et deviennent une feuille épidermique dans un récipient de culture de tissus, qui est retirée de la paroi du récipient en collant la feuille sur un élément de transport.

7. Procédé suivant la revendication 6, caractérisé en ce que la feuille épidermique est collée sur le côté derme d'une peau de porc.